# EUROPEAN PATENT APPLICATION

(11) **EP 4 789 678 A1**
(43) Date of publication of application: **12.08.2026**
(21) Application number: 25157227.7
(22) Date of filing: 11.02.2025
(51) Int. Cl.: A61K 38/05, A61P 13/04, A61K 31/43

(54) **ED PEPTIDE, OR A PHARMACEUTICALLY ACCEPTABLE SALT THEREOF, FOR TREATING UROLITHIASIS**

(71) Applicant: Capsa NMLplus GmbH, 7000 Chur (CH)
(72) Inventor: DUDKOV, Alexander, 1204 GENEVA (CH); HUBER, Daniel, 1248 HERMANCE (CH)
(74) Representative: Santarelli

(57) **Abstract**

The present invention relates to the field of therapeutic peptides for use in the treatment or the prevention of urolithiasis. In particular, the present invention pertains to ED peptide, or a pharmaceutically acceptable salt thereof, for use in the treatment or prevention of urolithiasis in a mammal. The present invention alose pertains to a pharmaceutical composition comprising ED peptide, and a product comprising ED peptide, or a pharmaceutically acceptable salt thereof, and an antibiotic as a combination product for simultaneous, separate or sequential use in the treatment or the prevention of urolithiasis.

## Description

### FIELD OF THE INVENTION

The present invention relates to the field of therapeutic peptides for use in the treatment or the prevention of urolithiasis.

### BACKGROUND OF THE INVENTION

Urolithiasis is a common pathology characterized by metabolic disorders, oxidative stress, hemodynamic disturbances, acidosis and necrosis of kidney tissue, followed by the formation and deposition of stones in the organs of the urinary system.

Current treatment regimens predominantly involve the use of antispasmodics, sedatives, analgesics, and antibiotics, supplemented by herbal medicines rich in flavonoids, tannins, vitamins, and silicic acid compounds for their salt-removing capabilities. Dietary adjustments and the use of feed supplements and enterosorbents further aid in optimizing mineral metabolism. In severe cases, particularly those involving urinary tract obstruction, surgical intervention such as lithotripsy is recommended.

The choice of therapy is highly dependent on the type of stone and the severity of the disease, representing a major therapeutic challenge. The lack of innovative, targeted therapies specifically designed for urolithiasis, in particular in veterinary medicine, represents a critical gap in the current approach.

### DETAILED DESCRIPTION

The present invention pertains to ED peptide, or a pharmaceutically acceptable salt thereof, for use in the treatment or prevention of urolithiasis in a mammal.

The ED peptide, as described herein, may be provided in isolated or purified form. An isolated form refers to a state in which the ED peptide is substantially separated from other components that are naturally associated with it, such as cellular components, other peptides, or proteins. A purified form refers to a higher level of separation, where the ED peptide has a purity of at least 90%, preferably at least 95%, 98%, or higher. These forms facilitate its use in pharmaceutical compositions for the treatment or prevention of urolithiasis in mammals.

The peptide according to the invention is an ultrashort peptide, such as an isolated or synthesized ultrashort peptide. As used herein, the term "ultrashort peptide" means a peptide consisting in two to seven amino acids.

Thus, the ED peptide according to the present invention is preferably a dipeptide L-Glu-L-Asp, or a pharmaceutically acceptable salt thereof. In the present specification, amino acid sequences are written from N-terminus to C-terminus unless otherwise stated. Advantageously, the peptide ED comprises L-amino acids, preferably consists in L-amino acids. Thus, according to a more preferred embodiment, the peptide according to the invention is a dipeptide having an amino acid sequence of ED, wherein the amino acids are L-amino acids, and wherein the amino acid sequence is written from N-terminus to C-terminus.

The peptide according to the invention, or the pharmaceutically acceptable salt thereof, can be synthesized by any convenient method, e.g. in the solid phase, the liquid phase or enzymatically. Furthermore, the peptide according to the invention can also be obtained from natural products by extraction.

As used herein, the terms "treatment" or "treating" and the like generally mean obtaining a desired pharmacological and physiological effect, leading to the inhibition and/or regretion of a disease. The effect may be therapeutic in terms of a partial or complete cure of a disease, condition, symptom or adverse effect attributed to the disease.

As used herein, the terms "prevention" or "preventing" and the like generally mean obtaining a prophylactic effect leading to the prevention or partial prevention of a disease, symptom or condition thereof. These terms can notably cover preventing the disease from occurring in a subject which may be predisposed to the disease but has not yet been diagnosed as having it such as a preventive early asymptomatic intervention.

In particular, the peptide according to the invention, or its pharmaceutically acceptable salt thereof, has low toxicity and gives low incidence of side effects.

As used herein, urolithiasis or kidney stone disease means a crystallopathy where a solid piece of material (renal calculus) develops in the urinary tract.

Urolithiasis may be induced by various causes such as high concentrations of certain substances in the urine (e.g., calcium, oxalate, uric acid and phosphate). Risk factors include dehydration, certain diets, and certain medical conditions such as hyperparathyroidism or frequent urinary tract infections, etc.

As mentioned above, the peptide according to the invention, or the pharmaceutically acceptable salt thereof, is used in the treatment or prevention in a mammal.

The peptide according to the invention, or the pharmaceutically acceptable salt thereof, may be administered to a mammal that suffer from urolithiasis.

Alternatively, the peptide according to the invention, or the pharmaceutically acceptable salt thereof, may be administered to a mammal which does not suffer from urolithiasis, for the sake of preventing urolithiasis (prophylaxie).

As used herein, the term "mammal" may refer to a human or an animal.

In a first embodiment, the mammal is a human.

Advantageously, according to this first embodiment, said peptide is administered at a dose from 0.01 µg/kg to 150 µg/kg per day.

In the context of the present application, and unless otherwise stipulated, the ranges of values indicated are understood to be inclusive.

Preferably, the peptide according to the invention, or the pharmaceutically acceptable salt thereof, may be administered to a human at a dose from 0.05 µg/kg to 100 µg/kg per day.

Such dosage can be adapted by the skilled person, in particular depending on whether the objective is treatment or prevention.

Unless otherwise specified, the expression "about X" means the value X ± 10%.

In a second embodiment, the mammal is an animal which may be a pet or a breeding animal, According to this embodiment, the use is thus a veterinary use.

Non limitative examples of breeding animals are horses, donkeys, cows, pigs, sheep, or goats.

Preferably, the mammal is a pet. Non limitative examples of pets are dogs, cats, rabbits, guinea pigs, hamsters, ferrets, gerbils, mice, or chinchillas, preferably dogs or cats Preferably, the pet to be treated with the peptide according to the invention is a cat or a dog, at any stage of life, such as: young cats, i.e. 1-6 years old cats, young dogs, i.e. 1-5 years old dogs, old cats, i.e. 12-14 years old cats, old dogs, i.e. 9-11 years old dogs.

Advantageously, ED peptide, or the pharmaceutically acceptable salt thereof, is administered at a dose from 0.01 µg/kg to 15 µg/kg per day.

Preferably, the peptide according to the invention, or the pharmaceutically acceptable salt thereof, may be administered to a pet, preferably a cat or a dog, at a dose from 0.05 µg/kg to 13 µg/kg per day. Still more preferably, the peptide according to the invention, or the pharmaceutically acceptable salt thereof, may be administered to a pet, preferably a cat or a dog, at a dose from 1 µg/kg to 10 µg/kg per day.

The present invention also relates to a pharmaceutical composition comprising ED peptide, or the pharmaceutically acceptable salt thereof, and an active principle for use in the treatment or prevention of urolithiasis in a mammal.

ED peptide is as described above, including advantageous and preferential features.

Advantageously, the ED peptide and the active principle in the pharmaceutical composition are present at effective dosages, either therapeutically effective or prophylactically effective.

Preferably, said active principle may be useful for treating any urinary diseases, in particular urolithiasis. For example, such active principle useful for treating any urinary diseases, in particular urolithiasis, may reduce urinary calcium content, reduce uric acid content, alkalinize urine, an analgesic effect, an antibiotic effect and/or an anti-inflammatory effect. Non limitative examples of such active principle useful for treating urolithiasis are thiazide diuretics (such as hydrochlorothiazide), allopurinol, potassium citrate, amoxicillin/sodium clavulanate or tiopronine.

In a first embodiment of the pharmaceutical composition, the active principle is an antibiotic. Preferably, the antibiotic can be selected among the group consisting of amoxicilin and amoxicillin/sodium clavulanate. In this embodiment, the pharmaceutical composition comprises ED peptide, or the pharmaceutically acceptable salt thereof, and a antibiotic for use in the treatment or the prevention of urolithiasis in a mammal.

In a second embodiment of the pharmaceutical composition, the active principle is a plant-based active principle. In this embodiment, the pharmaceutical composition comprises ED peptide, or the pharmaceutically acceptable salt thereof, and a plant-based active principle for use in the treatment or prevention of urolithiasis in a mammal. Preferably, the plant-based active principle comprises one or more plants selected from the list consisting of *Arctostaphylos uva-ursi, Urtica dioica, Equisetum arvense, Rubus idaeus, Orthosiphon stamineus, Hibiscus sabdariffa, Salvia rosmarinus, Levisticum officinale* and *Centaurium erythraea Rafn.*

More preferably, the plant-based active principle comprises *Salvia rosmarinus, Levisticum officinale* and *Centaurium erythraea Rafn.*

Still more preferably, the pharmaceutical composition according to the invention comprises both the antibiotic and the plant-based active principle.

In particular, the pharmaceutical composition comprises ED peptide , or the pharmaceutically acceptable salt thereof, an antibiotic and a plant-based active principle for use in the treatment or the prevention of urolithiasis in a mammal. Advantageously, the plant-based active principle comprises one or more plants selected from the list consisting of *Arctostaphylos uva-ursi, Urtica dioica, Equisetum arvense, Rubus idaeus, Orthosiphon stamineus, Hibiscus sabdariffa, Salvia rosmarinus, Levisticum officinale* and *Centaurium erythraea Rafn,* more preferably, *Salvia rosmarinus, Levisticum officinale* and *Centaurium erythraea Rafn.*

Advantageously, said pharmaceutical composition for use in the treatment or prevention of urolithiasis in a mammal, comprises ED peptide , or a pharmaceutically acceptable salt thereof at a concentration of between 9% and 95% by weight, such as 85% by weight, the percentage being expressed in relation to the total weight of said pharmaceutical composition.

The present invention also relates to a product comprising ED peptide , or a pharmaceutically acceptable salt thereof, and an antibiotic as a combination product for simultaneous, separate or sequential use in the treatment or the prevention of urolithiasis.

Such a product is a kit-of-parts ED peptide is as described above, including advantageous and preferential features.

Preferably, the product according to the invention also comprises a plant-based active principle comprising one or more plants selected from the list consisting of *Arctostaphylos uva-ursi, Urtica dioica, Equisetum arvense, Rubus idaeus, Orthosiphon stamineus, Hibiscus sabdariffa, Salvia rosmarinus, Levisticum officinale* and *Centaurium erythraea Rafn,* more preferably, *Salvia rosmarinus, Levisticum officinale* and *Centaurium erythraea Rafn,* as a combination product for simultaneous, separate or sequential use in the treatment or the prevention of urolithiasis.

The peptide, or the pharmaceutically acceptable salt thereof, the pharmaceutical composition and the product according to the invention may further comprise pharmaceutically acceptable carriers. Pharmaceutically acceptable carriers are well known in the art. Non limitative examples of pharmaceutically acceptable carriers are aqueous solutions such as (sterile) water, physiologically buffered saline, other solvents or vehicles (glycols, glycerol, oils or injectable organic esters, alcohol, fats, waxes), and inert solids. A pharmaceutically acceptable carrier may further contain physiologically acceptable compounds that act for example to stabilize or to increase the absorption of the peptide according to the invention. Such physiologically acceptable compounds include, for example, carbohydrates, such as glucose, sucrose or dextrans, antioxidants, such as ascorbic acid or glutathione, chelating agents, low molecular weight proteins or other stabilizers or excipients. One skilled in the art would know that the choice of a pharmaceutically acceptable carrier, including a physiologically acceptable compound, depends, for example, on the route of administration of the composition. Pharmaceutically acceptable adjuvants, buffering agents, dispersing agents, and the like, may also be incorporated into the pharmaceutical compositions.

The peptide, or the pharmaceutically acceptable salt thereof, the pharmaceutical composition and the product according to the invention, can be under various forms such as liquid form or solid form.

Preferably, the peptide according to the invention, or the pharmaceutically acceptable salt thereof, the pharmaceutical composition and the product according to the invention, can be under solid form, such as powder, tablet or capsule. This solid form in particulary advantageous when administered orally. In particular, for pets, it can be added directly to the feed or, for human, it can be added into the food, as a food supplement.

Alternatively, the peptide according to the invention, or the pharmaceutically acceptable salt thereof, the pharmaceutical composition and the product according to the invention, can be under liquid form, such as an aqueous solution. This liquid form in particulary advantageous when administered intraperitoneally, intramusculary or orally. In particular, when administered orally, it can be formulated as a solution that can be applied under the tongue.

The peptide may also be formulated in the pharmaceutical composition or the product according to the invention in liposomes. Such liposomes can prepared from a suitable lipid, for example a phospholipid such as lecithin, sphingomyelin, phosphatidyl ethanolamine, phosphatydil serine, cholesterol, phosphatidic acid, dicetyl phosphate, stearylamine, etc. The liposome can possess either a multi-layered or mono-layered structure and can also encapsulate a stabilizer, buffering agent, etc. Furthermore, the liposome can be made up into capsule form.

The peptide according to the invention, or the pharmaceutically acceptable salt thereof, the pharmaceutical composition and the product according to the invention, may be administered to the mammal through various routes of administration, that may be local or systemic. Non limitative examples of routes of administrations are intravenous, intraperitoneal, subcutaneous, intramuscular injections or oral administration, preferably, the route of administration is intraperitoneal or oral.

Preferably, peptide according to the invention, or the pharmaceutically acceptable salt thereof, or the pharmaceutically acceptable salt thereof, is administered by intraperitoneal administration. Such route of administration allows a fast absorption and can be used to administer large volumes of fluid safely.

Alternatively, the peptide according to the invention, or the pharmaceutically acceptable salt thereof, or the pharmaceutically acceptable salt thereof, is administered orally.

All the preferential features and embodiments described above applies to the present product.

The present invention also concerns a method for the prophylactic and/or therapeutic treatment of urolithiasis in a mammal, comprising administering to the mammal a composition comprising ED peptide or the pharmaceutically acceptable salt thereof.

All the preferential features and embodiments described above applies to the present method.

The method for the prophylactic and/or therapeutic treatment of urolithiasis can be performed on human.

Advantageously, in that case, the peptide according to the invention, or the pharmaceutically acceptable salt thereof, can be administered at a dose comprised between 0.01 µg/kg and 150 µg/kg, preferably between 0.5 µg/kg and 100 µg/kg. Advantageously, the duration of the treatment can be from 1 to 45 days, preferably 10 to 30 days such as 10 to 15 days. The peptide according to the invention, or the pharmaceutically acceptable salt thereof, can be administered one or more time per day such as once daily, twice daily, etc. Advantageously, the administration is performed in the morning. The dose may be increased depending on the patient's condition and the stage of the disease and the duration of the treatment may be increased for example to about 45 days.

Alternatively, the method for the prophylactic and/or therapeutic treatment of urolithiasis can be performed on a pet or a breeding animal, preferably on a dog or cats.

Advantageously, in that case, the peptide according to the invention, or the pharmaceutically acceptable salt thereof, can be administreted at a dose comprised between 0.01 µg/kg and 15 µg/kg, preferably between 0.05 µg/kg and 13 µg/kg, such as between 1 µg/kg and 10 µg/kg. Advantageously, the duration of treatment can be from 1 to 45 days, preferably 5 to 30 days, such as about 10 days. The peptide according to the invention, or the pharmaceutically acceptable salt thereof, can be administered one or more time per day such as once daily, twice daily, etc, preferably once daily. Advantageously, the administration is performed in the morning.

Preferably, when performed on dogs or cats, the peptide according to the invention, or the pharmaceutically acceptable salt thereof, can be administreted at a dose comprised between 0.1 µg/kg and 10 µg/kg, with a duration of about 10 days, once daily, in the morning.

The present invention also discloses a method for the prophylactic and/or therapeutic treatment of urolithiasis in a mammal, comprising administering to the mammal ED peptide or the pharmaceutically acceptable salt thereof in combination with other active principle(s). Preferably, said other active principle(s) may be useful for treating any urinary diseases, in particular urolithiasis. Said other active principle(s) are described above. More particularly, ED peptide and the other principle(s) can be in the form of a pharmaceutical composition or a kit-of-parts as described above.

The administration can be performed as described above.

The following examples are provided for the sake of illustrations, and does not limit the scope of the invention.

### EXAMPLES

### Example 1: In vivo study of normalization of urinalysis parameters of ED peptide on cats and dogs

The effects of ED peptide on the normalization of urinalysis parameters have been investigated in order to establish its uroprotective effect on cats and dogs.

### Animal selection

For the study on cats, 32 young and castrated cats (1-6 years old) and 32 old and castrated cats (12-14 years old) were selected. For the study on dogs, 45 young dogs (1-5 years old) and 45 old dogs (9-11 years old) were selected.

All animals were selected because they were suspected of suffering from urolithiasis. In particular, it was observed in theses animals symptoms characteristic of urolithiasis such as pollakiuria (i.e., frequent urination : 63.7% of cases in dogs and 59.6% of cases in cats), hematuria (i.e., presence of erythrocytes in urine : 46.3% of cases in dogs and 49.5% of cases in cats), stranguria (i.e., difficulty in urinating) and periuria (urination outside the tray), ischuria (i.e., urinary retention : 30% of cases in dogs and 26% of cases in cats), impaired appetite (22.1% of cases in dogs and 19.9% of cases in cats), anxiety (15% of cases in both animal species).

Pollakiuria, hematuria, stranguria and periuria were observed in animals for an average of 1-5 days.

### Treatments

All animals were monitored in an experiment lasting an average of 30 days. During the first 4 days, symptomatic treatment was administered to all animals, including antispasmodic, haemostatic, anti-inflammatory and detoxifying drugs.

Animals of each categories of age were divided into two groups.
- Group A includes animals that received only ED peptide (0.1 µg/kg intraperitoneally, once daily for the first 10 days).
- Control group includes animals that have not been subjected to any treatment. Animals in the control group received saline solution injections in the same scheme as ED peptide in Group A.

### Data collection and analysis

Animal urine was collected by aspiration cystocentesis on days 1, 15 and 30 after the start of treatment. The collected urine was analyzed within 1 hour of collection.

The tests focused on urine pH, protein concentration, transitional epithelium cell counts, squamous epithelium cell count, kidney epithelium cell count, red blood cells count, leukocytes count, struvite, bacterial presence. All of these parameters are criterion to evaluate the effectiveness of treatment of urolithiasis.

An alkaline pH favors the crystallization of calcium- and phosphate-containing stones.

Urine pH-metry is a laboratory study aimed at determining the amount of hydrogen ions in urine and assessing its acidity. This study was conducted using bromothymol blue.

Protein concentration in urine was measured by spectrophotometry. If proteins are present in the urine sample, they form a complex together, and the absorption spectrum of the latter shifts to the 600 nm region. The higher the concentration of protein molecules in the urine, the greater the optical density of the reaction at a wavelength of 600 nm.

Transitional epithelial cells, squamous epithelial cells, kidney epithelium, red blood cells, were investigated by light microscopy.

High levels of leukocytes in the urine typically indicate an infection in the urinary system. The presence of leukocytes is determined by the light microscopy and by the method of dry chemistry. The test strips used in the dry chemistry method reveal the esterase activity of cells. Esterase is found in granulocytes, mainly neutrophils, and histiocytes. The test strips allow to identify not only whole cells, but also their fragments. Therefore this method is used in addition to microscopy.

Struvite are a normal finding in urine, at low levels. In some pets, however, these struvite come together to form an actual stone within the urinary tract. These stones can irritate the urinary tract and have the potential to cause a urinary obstruction (blockage). In general, struvite begin to form when the urine pH is above 7. Struvite in urine were assessed visually, in particular by light microscopy.

Bacteria presence in urine were assessed visually by light microscopy.

Statistical data processing included calculating the mean, standard deviation, and confidence interval for each groups in the Statistica software. Shapiro-Wilk test was used to confirm the normal distribution of data. Student's t test was used to estimate the differences between groups. The critical level of significance of the null hypothesis about no differences was taken equal to 0.05.

### Results

The results of urine analysis from young cats, old cats, young dogs and old dogs during urolithiasis treatment are presented in Table 1, Table 2, Table 3 and Table 4, respectively.

**Table 1: young cats**

| Parameter | Day | Control group | Group A |
|---|---|---|---|
| pH | 1 | 7.9 ±0.3 | 7.6 ±0.4 |
| | 15 | 8.0 ±0.4 | 7.1 ±0.3* |
| | 30 | 8.7 ±0.3 | 7.4 ±0.5 |
| Protein, g/l | 1 | 0.50±0.12 | 0.53±0.10 |
| | 15 | 0.66±0.21 | 0.30±0.09* |
| | 30 | 0.57±0.2 | 0.32±0.07* |
| Transitional epithelium, pcs/in FOV | 1 | 11±2 | 11±2 |
| | 15 | 10±2 | 7±0,3* |
| | 30 | 9±1 | 3±0,2* |
| Squamous epithelium, pcs/in FOV | 1 | 21±3 | 18±3 |
| | 15 | 23±4 | 8±2* |
| | 30 | 26±5 | 5±1* |
| Kidney epithelium, pcs/in FOV | 1 | 3±0,5 | 3±0.3 |
| | 15 | 4±0,5 | 1.5±0.3* |
| | 30 | 4±0,5 | - |
| Red blood cells, pcs/in FOV | 1 | 180±25 | 200±20 |
| | 15 | 160±22 | - |
| | 30 | 170±20 | - |
| Leukocytes, pcs/in FOV | 1 | 29±4 | 28±3 |
| | 15 | 27±3 | 25±3 |
| | 30 | 28±4 | 6±1* |
| Struvite | 1 | +++ | +++ |
| | 15 | +++ | ++ |
| | 30 | +++ | + |
| Bacteria | 1 | +++ | +++ |
| | 15 | +++ | ++ |
| | 30 | +++ | - |

| | | | |
|---|---|---|---|
| * p<0.05 compared to Control group | | | |

**Table 2: old cats**

| Parameter | Day | Control group | Group A |
|---|---|---|---|
| pH | 1 | 8.1 ±0.4 | 8.0±0.6 |
| | 15 | 8.0 ±0.5 | 6.5±0.5* |
| | 30 | 8.9±0.4 | 6.1±0.4* |
| Protein, g/l | 1 | 0.96±0.15 | 0.93±0.11 |
| | 15 | 0.66±0.20 | 0.58±0.03* |
| | 30 | 0.60±0.20 | 0.40±0.09* |
| Transitional epithelium, pcs/in FOV | 1 | 15±2 | 17±2 |
| | 15 | 12±2 | 8±0.8* |
| | 30 | 10±2 | 5±0.6* |
| Squamous epithelium, pcs/in FOV | 1 | 20±3 | 23±3 |
| | 15 | 20±4 | 16±2* |
| | 30 | 20±3 | 9±2* |
| Kidney epithelium, pcs/in FOV | 1 | 3±0.5 | 6±1 |
| | 15 | 3±0.3 | 3±0.3 |
| | 30 | 3±0.5 | 2±0.2* |
| Red blood cells, pcs/in FOV | 1 | 220±20 | 215±20 |
| | 15 | 190±20 | - |
| | 30 | 180±25 | - |
| Leukocytes, pcs/in FOV | 1 | 33±4 | 32±3 |
| | 15 | 30±4 | 20±3* |
| | 30 | 30±4 | 12±2* |
| Struvite | 1 | +++ | +++ |
| | 15 | +++ | + |
| | 30 | +++ | - |
| Bacteria | 1 | +++ | +++ |
| | 15 | +++ | ++ |
| | 30 | +++ | - |

| | | | |
|---|---|---|---|
| * p<0.05 compared to Control group | | | |

**Table 3: young dogs**

| Parameter | Day | Control group | Group A |
|---|---|---|---|
| pH | 1 | 8.8 ±0.8 | 8.8 ±1.0 |
| | 15 | 8.5 ±1.3 | 6.6 ±0.9* |
| | 30 | 8.9 ±0.9 | 6.2 ±0.8* |
| Protein, g/l | 1 | 0.55±0.10 | 0.54±0.10 |
| | 15 | 0.59±0.12 | 0.33±0.09* |
| | 30 | 0.95±0.15 | 0.26±0.05* |
| Transitional epithelium, pcs/in FOV | 1 | 13±2 | 13±2 |
| | 15 | 13±2 | 6±0.4* |
| | 30 | 11±1 | 4±0.5* |
| Squamous epithelium, pcs/in FOV | 1 | 20±3 | 21±3 |
| | 15 | 22±4 | 13±2* |
| | 30 | 23±4 | 7±2* |
| Kidney epithelium, pcs/in FOV | 1 | 3±0.5 | 3±0.2 |
| | 15 | 3±0.5 | 3±0.2 |
| | 30 | 3±0.5 | - |
| Red blood cells, pcs/in FOV | 1 | 195±22 | 198±20 |
| | 15 | 190±20 | - |
| | 30 | 185±20 | - |
| Leukocytes, pcs/in FOV | 1 | 25±3 | 25±3 |
| | 15 | 24±3 | 16±2* |
| | 30 | 25±4 | 7±1* |
| Struvite | 1 | +++ | +++ |
| | 15 | +++ | + |
| | 30 | +++ | - |
| Bacteria | 1 | +++ | +++ |
| | 15 | +++ | + |
| | 30 | ++ | - |

| | | | |
|---|---|---|---|
| * p<0.05 compared to control group | | | |

**Table 4: old dogs**

| Parameter | Day | Control group | Group A |
|---|---|---|---|
| pH | 1 | 9.2 ±0.9 | 9.0 ±1.0 |
| | 15 | 9.5 ±1.0 | 6.7 ±0.7* |
| | 30 | 9.5 ±0.9 | 5.9±0.7* |
| Protein, g/l | 1 | 0.60±0.10 | 0.64±0.12 |
| | 15 | 0.62±0.13 | 0.39±0.09* |
| | 30 | 0.66±0.15 | 0.28±0.07* |
| Transitional epithelium, pcs/in FOV | 1 | 10±2 | 10±2 |
| | 15 | 10±2 | 9±2 |
| | 30 | 10±2 | 6±1* |
| Squamous epithelium, pcs/in FOV | 1 | 22±4 | 22±3 |
| | 15 | 22±3 | 17±2* |
| | 30 | 20±4 | 7±1* |
| Kidney epithelium, pcs/in FOV | 1 | 4±1 | 4±0,5 |
| | 15 | 3±1 | 4±0,5 |
| | 30 | 3.5±0.5 | 2±0,5* |
| Red blood cells, pcs/in FOV | 1 | 220±20 | 210±20 |
| | 15 | 215±22 | - |
| | 30 | 220±20 | - |
| Leukocytes, pcs/in FOV | 1 | 36±4 | 35±5 |
| | 15 | 30±5 | 19±3* |
| | 30 | 32±4 | 9±2* |
| Struvite | 1 | +++ | +++ |
| | 15 | +++ | ++ |
| | 30 | +++ | + |
| Bacteria | 1 | +++ | +++ |
| | 15 | +++ | + |
| | 30 | ++ | - |

| | | | |
|---|---|---|---|
| * p<0.05 compared to Control group | | | |

The results from Tables 1-4 show that when ED peptide is administered in cats or dogs, all urine parameters are significantly improved compared to the control group.

Consequently, results demonstrate an effective *in vivo* therapeutic effect of ED peptide on urolithiasis in mammals, such as cats and dogs.

### Example 2: In vivo study of normalization of urinalysis parameters of ED peptide in combination with standard therapy on cats and dogs

The effects of ED peptide in combination with standard therapy on the normalization of urinalysis parameters have been investigated in order to establish its curative and uroprotective effect on cats and dogs.

### Animal selection:

Animal selection is the same as in Example 1.

### Treatments

All animals were monitored in an experiment lasting an average of 30 days. During the first 4 days, symptomatic treatment was administreted to all animals , including antispasmodic, haemostatic, anti-inflammatory and detoxifying drugs.

Animals of each categories of age were divided into four groups.
- Group 1 includes animals that received standard treatment for urolithiasis: amoxicillin/sodium clavulanate as an antibiotic (12.5 µg/kg, twice daily for the first 14 days), and Canephron orally (1/3 tablet, twice daily for 30 days).
- Group 2 includes animals that received standard treatment for urolithiasis: amoxicillin/sodium clavulanate as an antibiotic (12.5 µg/kg, twice daily for the first 14 days), and Canephron orally (1/3 tablet, twice daily for 30 days), and ED peptide (0.1 µg/kg intraperitoneally, once daily for the first 10 days).
- Group 3 includes animals that received only ED peptide (0.1 µg/kg intraperitoneally, once daily for the first 10 days).
- Control group includes animals that have not been subjected to any treatment. Animals in the control group received saline solution injections in the same scheme as ED peptide in Group 3.

### Data collection and analysis

Animal urine samples were collected and analysed following the procedure outlined in Example 1:
- Urine pH for young cats was measured on day 30,
- Leukocyte counts for young cats were assessed on day 15;
- Protein concentration, squamous epithelial cells and kidney epithelial cells for old cats, were measured on day 15.
- Protein concentration for young dogs was measured on day 15.

### Results

The results of urine analysis from youngs cats, old cats and young dogs during urolithiasis treatment are presented in Table 5.

**Table 5: young cats, old cats and young dogs**

| **Animals** | **Parameters** | **Control group** | **Group 1** | **Group 2** | **Group 3** |
|---|---|---|---|---|---|
| Young cats | pH at 30^{th} day | 8.7 ±0.3 | 6.9 ±0.4* | 5.2 ±0.4*, ** | 7.4 ±0.5 |
| | % of decrease with respect to the control group | | 20.7% | 40% | 15% |
| | Leukocytes, pcs/in FOV at 15th day | 27±3 | 13±2* | 9±1*, ** | 25±3 |
| | % of decrease with respect to the control group | | 51.8% | 66.6% | 7.4% |
| Old cats | Protein, g/l at 15th day | 0.66±0.20 | 0.55±0.0* | 0.30±0.04*,** | 0.58±0.03* |
| | % of decrease with respect to the control group | | 16.6% | 54.5% | 12% |
| | Squamous epithelium, pcs/in FOV at 15th day | 20±4 | 12±2* | 7±1* | 16±2* |
| | % of decrease with respect to the control group | | 40% | 65% | 20% |
| | Kidney epithelium, pcs/in FOV at 15th day | 3±0.3 | 2±0.3* | 1±0.2*,** | 3±0.3 |
| | % of decrease with respect to the control group | | 33% | 66% | 0% |
| Young dogs | Protein, g/l at 15th day | 0.59±0.12 | 0.45±0.10* | 0.20±0.03*,** | 0.33±0.09* |
| | % of decrease with respect to the control group | | 23.7% | 66% | 44% |

| | | | | | |
|---|---|---|---|---|---|
| * p<0.05 compared to Control group ** p<0.05 compared to Group 1 | | | | | |

The results from Table 5 demonstrate that, when combined with the ED peptide, standard (antibiotic) treatment resulted in a synergistic effect on most of the parameters of interest. Daily intraperitoneally administration of a dose of 0.1 µg/kg of ED peptide in cats or dogs induces an improvement in the well-being of these animals suffering from urolithiasis.

## Claims

1. ED peptide, or a pharmaceutically acceptable salt thereof, for use in the treatment or prevention of urolithiasis in a mammal.

2. The peptide for use of claim 1, wherein said peptide is administered to a human.

3. The peptide for use of claim 2, wherein said peptide is administered at a dose from 0.01 µg/kg to 150 µg/kg per day.

4. The peptide for use of claim 1, wherein said peptide is administered to a pet.

5. The peptide for use of claim 4, wherein said peptide, or the pharmaceutically acceptable salt thereof, is administered at a dose from 0.01 µg/kg to 15 µg/kg per day.

6. A pharmaceutical composition comprising ED peptide, or the pharmaceutically acceptable salt thereof, and an active principle for use in the treatment or prevention of urolithiasis in a mammal.

7. A product comprising ED peptide, or a pharmaceutically acceptable salt thereof, and an antibiotic as a combination product for simultaneous, separate or sequential use in the treatment or the prevention of urolithiasis.
